# EUROPEAN PATENT SPECIFICATION

(11) **EP 1 497 652 B1**
(45) Date of publication and mention of the grant of the patent: **14.11.2007**
(21) Application number: 03746394.0
(22) Date of filing: 27.03.2003
(51) Int. Cl.: G01N 33/543

(54) **METHOD, SYSTEM AND KIT FOR DETECTING AN ANALYTE IN A SAMPLE**
VERFAHREN, SYSTEM UND KIT ZUM NACHWEIS EINES ANALYTEN IN EINER PROBE
METHODE, SYSTEME ET KIT PERMETTANT DE DETECTER UN ANALYTE DANS UN ECHANTILLON

(30) Priority: 16.04.2002 US 372432 P
(43) Date of publication of application: 19.01.2005
(73) Proprietor: Matis Medical Inc., Sunrise, FL 33351 (US)
(72) Inventor: VARON, David, 76965 Kfar Bilu A (IL)
(74) Representative: Jennings, Tara Romaine
(86) International application number: PCT/IL2003/000257
(87) International publication number: WO 2003/087825

(56) References cited:
- WO-A-95/04930
- WO-A-96/12966
- US-A- 3 770 380
- US-A- 4 692 412
- US-A- 5 487 112
- US-A- 5 541 417

## Description

### FIELD OF THE INVENTION

The present invention concerns detection of analytes in fluid samples by formation of a thin layer of aggregates comprising the analyte, and analysis of the aggregates thus formed, preferably by the use of image analysis.

### BACKGROUND OF THE INVENTION

Early detection of an active disease in a patient is an essential factor for a successful treatment. Provided that a rapid and correct diagnosis is established, it is possible to slow down the progress and at times cure patients from a disease.

Traditional methods for the detection of infectious diseases include serology assays, e.g. complement fixation (CF), indirect and direct fluorescent antibody (IFA and DFA, respectively), enzyme-linked immunosorbent assay (ELISA) and latex agglutination; culturing assays in which the infectious microorganism recovered from a patient during acute infection is cultured and then identified; and assays involving the use of monoclonal antibodies specific against the infectious agent.

Flow cytometry analysis is also used for disease detection and involves the measuring of certain physical and chemical characteristics of cells or particles, including cell size, shape and internal complexity or any other cell component that can be detected by a fluorescent compound, as the cells or particles travel in suspension one by one past a sensing point. The use of flow cytometry for detection methods has been described, for example in WO99/47933. This publication describes a method for the detection of surface antigens by contacting an antibody-coupled bead with a test sample and, if the target antigen is present in the sample, a bead-antibody-antigen complex is thus formed and detected by flow cytometry.

US 6,159,748 describes a kit for the detection of antibodies in serum samples using a flow cytometer. In particular, the kit is provided with beads coated with a series of antigens, each having a different bead size and carrying a different antigens. The beads are used for the detection of different antibodies, including auto-antibodies.

As appreciated by those versed in the art, when utilizing a flow cytometry instrument, the cell sample preparation, data collection and data analysis must be performed by a specially trained technician. The flow cytometry instrument includes a laser and complex optical system, a high-power computer and electrical and fluidic systems. The component systems of the flow cytometry instrument must be properly maintained and calibrated on a regular and frequent basis. The high cost of the instrument and the expertise required to correctly operate such instrument render detection by flow cytometry convoluted and expensive. Evidently, this rational also applies to many other tests and instruments, including, *inter alia,* Enzyme-Linked Immunosorbent Assay (ELISA).

WO 01/33215 and WO 02/79749 describe systems for generating a profile of particulate components of a body fluid sample. The systems include in general a device for causing controlled flow of the body fluid sample on a substrate, the controlled flow of the body fluid sample leading to a differential distribution of the particulate component on the substrate, and a magnifying device being for providing a magnified image of differentially distributed particulate components on the substrate. The magnified image represents a profile of the particulate components of the body fluid sample. The systems described may further comprise an image analyzer for analyzing the profile of the particulate component in the body fluid sample.

US Patent Specification No. US-A-5,541,417 discloses methods of analysis of a reaction, such as an agglutination reaction producing an agglutination pattern. In one method, an image of the agglutination pattern is captured. The image captured is digitized to form a digital image comprising a pixel. Roughness is measured, reflecting pixel local environment, at each pixel comprising a region of interest of the digital image of the agglutination pattern. At least one of classification and quantification of the image captured of the agglutination pattern is performed based on the measured roughness.

### SUMMARY OF THE INVENTION

The present invention aims for providing a rapid, sensitive and easy-to-perform method of detecting *in vitro* analytes in a fluid sample making use of an optical image analyzer. The method of the invention is preferably aimed for therapeutic diagnosis, however, may be suitable for other applications, e.g. ecological, environmental, etc.

Thus, according to a first of its aspects, the present invention provides a method for detecting an analyte in a fluid sample comprising:
(a) mixing said fluid sample with a reagent comprising a capturing agent which is a first member of a binding couple that can bind to an analyte, the analyte being a second member of the binding couple, such that if the analyte is present in the fluid sample, particulates of the binding couple are formed;
(b) treating said mixture so as to form on a solid substrate a monolayer of said particulates, if formed as a result of said mixing;
(b) obtaining an optical image of the monolayer; and
(c) analyzing said image so as to determine therefrom the absence or presence of particulates formed as a result of the association between the binding couple, the presence of particulates in the sample indicating the presence of said analyte in the sample; or to determine therefrom at least one parameter of said particulates.

A parameter according to the invention may be, without being limited thereto: size of particulates or size distribution of the particulates formed as a result of the association between the binding couple; particulates' count; particulates shape; and/or spatial distribution of the formed particulates.

A system for performing the method of the invention is disclosed, the system comprising:
(a) holding means for holding a solid substrate carrying a thin layer of particulates;
(b) an optical image acquisition device for capturing an image of the thin layer on the solid substrate;
(c) an image analysis device coupled to said image acquisition device and for analyzing an image obtained by the image acquisition device.

The system optionally comprises a magnifying device.

Yet further, a kit for use in the method of the invention is disclosed, the kit comprising:
(a) at least one reagent comprising a capturing agent being a first member of a binding couple and comprising at least two capturing moieties to which binds an analyte if present in a fluid sample, the analyte being a second member of the binding couple; and
(b) a solid substrate for carrying a thin layer of particulates.

### BRIEF DESCRIPTION OF THE FIGURES

In order to understand the invention and to see how it may be carried out in practice, some embodiments will now be described, by way of non-limiting examples only, with reference to the accompanying Figures, in which:
**Figs. 1A-1C** show light microscope images of plasma samples incubated with microbeads coated with multiple copies of an antibody directed against D-dimer. The plasma samples containing a very low level of D-dimer **(Fig. 1A),** an intermediate level of D-dimer **(Fig. 1B)** or a high level of D-dimer **(Fig. 1C).**
**Fig. 2A-2C** are bar representations of the size distribution of particulates formed as a result of binding of D-dimer to microbeads coated with antibodies directed against D-dimer. Microbeads coated with multiple copies of antibodies directed against D-dimer were incubated with samples containing low levels of D-dimer (Fig. 2A), intermediate levels of D-dimer (Fig. 2B) or high levels of D-dimer (Fig 2C).
**Fig. 3** shows the average size of particulates obtained as a result of titration of plasma samples containing different concentrations of D-Dimer.
**Fig. 4** shows the average size of particulates obtained with heparin induced thrombocytopenia (HIT) samples by use of the Diamed kit with negative samples (n=4) as well as positive samples (n=10).
**Fig. 5A-5B** show light microscope images of EDTA-anticoagulated type A blood samples mixed with a dilution buffer (0.9 % NaCl and 2% bovine albumine) to give a final dilution of 100-fold and anti blood group A (Fig. 5A) or anti blood group B (Fig. 5B) antibodies are added to a final concentration of 0.1 to 0.25 mg/mL, the mixture is than incubated for 1 min with gentle mixing followed by examination by light microscope. Fig. 5B shows an image of a negative response while Fig. 5A shows an image of a positive response.
**Fig. 6** is a graph showing the average size of blood aggregates of blood groups O, A and AB incubated with antibodies against A or B groups obtained and determined by performing the method of the invention.

### DETAILED DESCRIPTION OF THE INVENTION

The present invention provides a rapid and easy *in vitro* method of detecting an analyte in, preferably, a biological fluid sample obtained from a subject, without the need of sophisticated equipment or professional skills to analyze the sample. Moreover, the method of the invention is sensitive and allows detection at an early stage of disease and provides a tool to follow a patient from onset to the recovery from a specific disease and to monitor the effectiveness of a chosen treatment against a disease. The sensitivity of the method of the invention arises from the creation of a thin layer of the particulates formed in the analyzed sample (after being mixed with a suitable reagent), if the analyte is present in the sample. The formation of a thin layer enables the accurate image analysis of the particulates so as to obtain a qualitative as well as a quantitative determination with respect to the analyte.

It is to be understood that both the forgoing general description and the following description of some specific embodiments of the invention have been provided merely for the purpose of explanation and are in no way to be construed as limiting the present invention as claimed.

Thus, according to one aspect of the invention there is provided a method for detecting an analyte in a fluid sample, the method comprising:
(a) mixing said fluid sample with a reagent comprising a capturing agent which is a first member of a binding couple that can bind to an analyte, the analyte being a second member of the binding couple, such that if the analyte is present in the fluid sample, particulates of binding couples are formed;
(b) treating said mixture so as to form on a solid substrate a monolayer of said particulates, if formed as a result of said mixing;
(c) obtaining an optical image of the monolayer; and
(d) analyzing said optical image so as to determine therefrom the absence or presence of particulates formed as a result of the association between the binding couple, the presence of particulates in the sample indicating the presence of said analyte in the sample; or to determine from said image at least one parameter of said particulates.

The term ***"detect* "or *"detection*** "as used herein refers collectively to both a qualitative and quantitative determination of the presence of an *analyte* in a sample.

Thus, the method of the present invention also provides analytical (quantitative) detection of a target analyte in a fluid sample. According to this embodiment, i.e. the quantitative detection, one or more parameters characterizing the particulates formed as a result of association of the binding couple is determined. Such parameters can be easily defined by a man skilled in the art, and include, for example determination of the size of the particulates formed as a result of association between the first and second members of the binding couple, size distribution of the particulates, the number of particulates formed (particulate count), the pattern of distribution, etc. In order to analytically determine the above parameters, it is essential that a thin layer of the fluid sample and reagent is formed, as explained hereinafter.

According to the invention, the association (binding or complexing) between an *analyte* to a respective *capturing agent* results in the formation of a complex. The capturing agent and the analyte constitute together a binding couple. The binding couple may, for example, be one of the couples selected from the group of receptor-ligand, sugar-lectin, antibody-antigen (the term *"antibody"* should be understood as referring to a polyclonal or a monoclonal antibody, to a fraction of an antibody comprising the variable, antigen-biotin binding portion, etc.).

The term *"analyte",* according to a first aspect of the invention, refers to a cellular or microorganism component such as proteins (e.g. antibodies, cytokines, receptors), glycoproteins, peptides, low molecular weight compounds, the detection of which in a sample obtained from a subject is indicative of whether the subject has a specific disease or disorder The term *"analyte"* may refer also to a synthetic or natural chemical, or a drug or a toxin. The analyte according to this aspect of the invention contains at least two binding sites (recognition sites) to which two individual and separate capturing agents may bind. The results of binding to each binding site of the analyte to an individual capturing agent thus results in the formation of aggregates of binding couples (referred to herein also by the term *"particulates").*

According to another embodiment, the analyte refers to particles presenting on their surface at least two binding (recognition) sites. For example, the analyte may include antigen-presenting particles, e.g. antigen presenting cells, viruses or other infectious microorganisms, which present on their surface more than one copy of a specific antigen to which the capturing agent binds.

The term *"capturing agent"* according to the invention refers to any bi or multifunctional agent, which can bind, preferably with specificity, to two or more analytes in a sample, thereby forming aggregates of binding couples. Accordingly; the capturing agent includes dimmeric, trimeric or multimeric molecules presenting, respectively, two, three or more capturing sites which can bind independently to an analyte in the fluid sample. For example, the agent may be a dipeptide or diprotein bridged by a linker. According to a preferred embodiment, the capturing agents are microbeads coated with specific capturing moieties. The capturing agent comprises a *"capturing moiety"* which is, in principle, a binding site which the analyte has an affinity and the association between the two is as a result of association between the said capturing moiety (site) of the capturing agent and the recognition site of the analyte. The meaning of the above terms may be further understood in view of the following non-limiting examples.

According to one embodiment of the invention, the capturing agent is a particle comprising at least two epitopes and the analyte is an antibody (comprising two binding sites) to which antigenic epitopes of different capturing agents binds, or *vice versa,* the agent is an antibody (comprising two recognition sites) and the analyte is an antigen comprising at least two antigenic epitopes or a particle presenting on its surface at least two antigenic epitopes to which two or more antibodies can bind.

According to a second aspect of the methods of the invention, the capturing agents are microbeads coated with capturing moieties. The microbeads may comprise on their *sensing interface* a single type of capturing agent or several types of capturing agents so as to enable the use of the coated microbeads in different detection assays. The *"sensing interface"* refers preferably to the outer surface of the beads, which is coated with the capturing agent(s) so as to allow the formation of the resulting particulates.

Microbeads which are used according to the invention may be made of polymer such as polystyrene, latex etc., which are coated with the capturing agent either by simple adsorption, by the aid of cross-linking agents or any other method of conjugating the capturing agent to the microbeads, as known by those versed in the art. The microbeads according to the invention may also be referred to as *affinity beads* and according to one embodiment the microbeads are immunobeads.

The *sample* according to the invention refers preferably to a fluid biological sample and more preferably to any body fluid, including blood (plasma and serum), saliva, urine or cellular moieties derived from body fluids (e.g. blood cells), or cellular components which may be obtained from a tissue or from body cavities and then suspended in a suitable medium for detection by the method of the present invention. Nevertheless, the sample according to the invention may also be of other sources, e.g. for the detection of analytes in sewages, water reservoirs, chemical solutions, etc. Therefore, while the following examples refer to biological samples, the invention should be construed as applying also to detection of analytes in non-biological samples, such as chemicals.

The optical image obtained may be a magnified image of the thin layer of the sample and the magnification can be achieved by the use of a light microscope lens.

The light microscope lens may be constructed within a light microscope device, or within any other technical means known in the art for optically viewing a micro-image within a sample. The light microscope lens may be coupled to an optical image acquisition device.

The term *"associate"* or *"association"* as used herein in connection with the capturing agent and recognition site on the analyte (so as to form the binding couple) refers to any form of combination between the first and second member of the binding couple, which results in the formation of the optically detectable particulate matter comprised of the binding couple. The term *"associate"* thus includes all types of chemical bonding, e.g. ionic bond, covalent bond, metallic bond, hydrogen bond, *Van der Waals* bond and electric dipoles. Thus, the -association between the binding couple may be a strong association (e.g. in case of covalent bonding) or a week association (e.g. hydrogen bond) and in any case the association is sufficiently stable to allow the imaging of the particulate formed.

According to the method of the invention it is essential that a monolayer of the particulate matter obtained from the mixture of the analyte in the fluid sample and the reagent is formed on a solid substrate, so as to enable the optical imaging and analysis of particulate matter formed within the fluid sample, in case the analyte is present in said sample.

A *"thin layer"* refers to *a substantially uniform layer* of aggregates/particulates of binding couples formed as a result of association between the capturing agent (the first member of the binding couple) and the analyte (the second member of the binding couple). *A substantially uniform layer* means that there is essentially no overlaying of one binding couple (or particulate comprising binding couples) on top of another binding couple (or particulate comprising binding couples) and that there is substantially only one (single) particulate/object/aggregate at the vertical dimension of the layer. According to the invention, a thin layer is a monolayer.

There are several methods of obtaining a substantially uniform layer or monolayer of particulates, such as those formed according to the instant invention. For example, a thin layer of particulates may be obtained by fixation of the particulates to the solid substrate, e.g. by saturating the solid substrate carrying the sample-reagent mixture with a spray fixative or by immersion of the mixture with a suitable fixative solution; by the use of capturing agents immobilized to the solid substrate; by the use of high specific gravity capturing agents (e.g. high specific gravity beads coated with capturing moieties that precipitate by gravity force to the bottom of the testing chamber); by the use of magnetic capturing agents (e.g. magnetic beads coated with capturing moieties); by applying mechanical pressure onto the sample-capturing agent mixture (e.g. by applying a solid cover); by the use of a *Cytospin* technology which uses centrifugal force to separate and deposit a monolayer of a substance, typically cells, on slides while maintaining the substance's integrity; etc.

In cases a binding couple is formed and a thin layer of binding-couple particulate matter is created, the method of the invention may include the additional step of separating the thin layer from the fluid carrier. Methods of separating thin layers from fluid carriers have been developed, e.g. by LaMina, Inc. (Arlington, VA, e.g. in US patent No. 6,423,237; 6,106,483; 6,091,483 and others.)

A system for performing the method of the invention is disclosed, the system comprising:
(a) holding means for holding a solid substrate carrying a thin layer of particulates;
(b) an optical image acquisition device for capturing an image of the thin layer on the solid substrate;
(c) an image analysis device coupled to said image acquisition device and for analyzing an image obtained by the image acquisition device.

The system may further comprise a magnifying device. According to one preferred embodiment, the magnifying device comprises light microscope lenses and according to a more preferred embodiment, the magnifying device is a light microscope.

The optical image acquisition device may be any such device known in the art of optical imaging, however, is preferably a camera. The image acquisition device is coupled to said magnifying device if the latter is present.

According to one embodiment, analysis of the image includes determination of one or more parameters indicative of the presences and concentration of the analyte in the sample, the parameter may be selected from: size distribution of particulates formed as a result of interaction between the analyte in the sample and the capturing agent; number of particulates formed as a result of said interaction; shape of said particulates; and/or spatial distribution of the formed particulates.

The system is optionally equipped with a solid substrate. The *solid substrate* according to the invention may include any carrier for carrying the sample subject of detection and on which the association between the reagent comprising capturing agent and the analyte, if present in the sample, may be performed. The solid substrate is designed such that a thin layer of particulates of the binding couple may be formed thereon. The solid substrate thus may be, without being limited thereto, a microscope slide, or a testing chamber. In this connection, it should be understood that the mixing of the fluid sample and the reagent may be performed in a different carrier and a thin layer of particulates formed may then be transferred to the solid substrate for analysis.

Alternatively, the solid substrate may be a container at the bottom of which the particulates are accumulated in the form of a thin layer.

Optical image acquisition devices (imaging sensors) are well known in the art and thus should not be further detailed. One example of a device includes video cameras (e.g. CCD or CMOS Camera), which may be mounted on the microscope. The images obtained can be sent to a data processing unit and be analyzed by any known image analysis software (e.g. an image analysis software developed by Galai, Beit- Haemek, Israel or a specifically designed software) to determine the number of aggregates and the distribution of the particulate sizes formed as a result of aggregation. The distribution of the particulate size correlated with the concentration of the analyte in the tested specimen and with the number of complexes formed between capturing agents and analytes as a result of incubation.

A kit for use in the method of the present invention is disclosed comprising:
(a) at least one reagent comprising a capturing agent being a first member of a binding couple and comprising at least two capturing moieties to which binds an analyte, if present in a tested fluid sample, the analyte being a second member of the binding couple,
(b) a solid substrate for carrying a thin layer of particulates.

The kit may further comprise means for creating a thin layer of the mixture comprising the fluid sample and the capturing agent. These means depend on the type of solid substrate and/or capturing reagent employed. For example, with a microscope slide or a testing chamber serving as a solid substrate, the thin layer may be created by applying a cover slide onto the sample-reagent mixture. The pressure applied onto the fluid sample thus causes the formation of a thin layer of the latter. Alternatively, the kit may comprise fixation reagents for fixating/immobilizing the capturing agent onto the solid substrate in a thin layer structure. Further, in case the capturing agent is comprised of magnetic substance, they may be arranged in a thin layer by the use of magnetic forces.

The invention will now be illustrated by the following non-limiting Examples with reference to the attached figures.

### SPECIFIC EXAMPLES

### Example 1

The following examples were performed using Latex-microbeads coated with an antibody directed against D-dimer (Biopool International, Umea Sweeden, Cat# 150709, Example 1.1) or polymer beads, coated with heparin/PF4 complexes (DiaMed-ID PaGIA [Particle Gel Immuno Assay], Cat # 050051, DiaMed AG, 1785 Cressier s/Morat, Switzerland, Example 1.2).

In general, plasma samples were incubated with microbeads coated with the specific capturing agents for a predetermined time period. After incubation, each sample was covered to form a thin layer of the mixture and placed on a light microscope slide and examined by a light microscope. Images of the resulting thin layer of the samples were captured by a video camera (CCD Camera) mounted on the microscope. The images thus obtained were analyzed by an image analysis software (Galai, Beit- Haemek, Israel), to determine the number of aggregates and the distribution of the particulate sizes formed as a result of aggregation. The distribution of the particulate size correlated with the concentration of the analyte in the tested specimen and with the number of complexes formed between capturing agents and analytes as a result of incubation.

### Example 1.1

A D-dimer kit (Dade Behring Inc.) was used in order to determine the presence of D-dimer in plasma samples and operated according to manufacturer's instructions. In this specific assay three plasma samples: (i) containing a very low level of D-dimer, (ii) containing an intermediate level of D-dimer or (iii) containing a high level of D-dimer were tested for the presence of D-dimer by the use of microbeads coated with antibodies directed against D-dimer. The microbeads were incubated with each sample for 1 minute, after which the samples covered with a cover-slide (to form a thin layer of the mixture) and transferred to microscope plates and analyzed as described above.

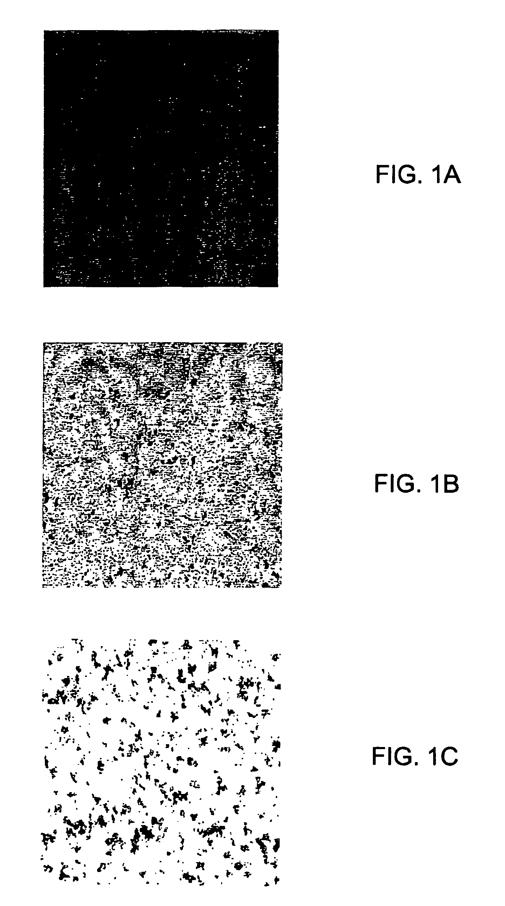

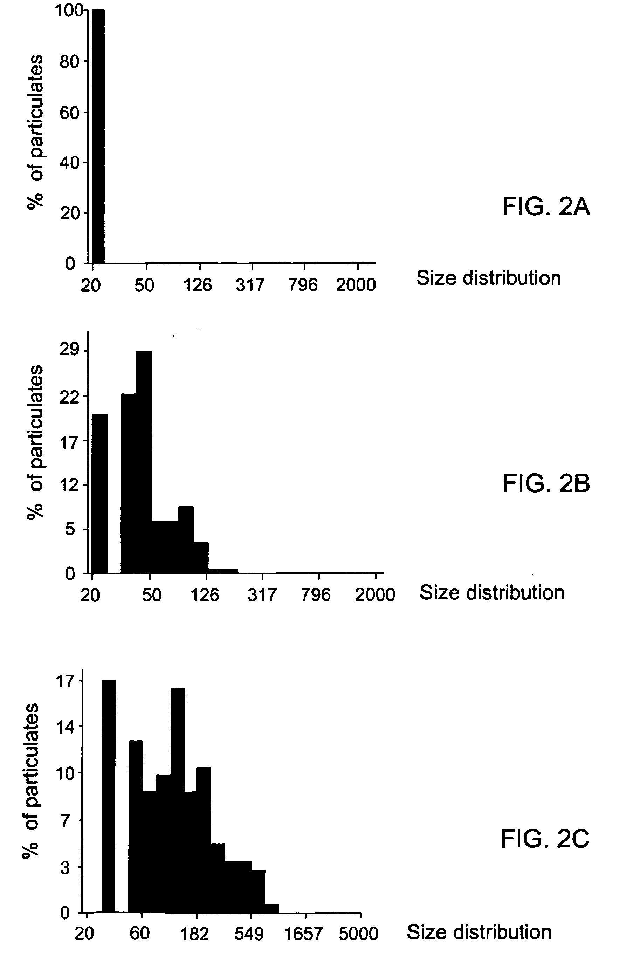

Figs. 1A-1C and 2A-2C show the results obtained. In particular, a microscope specimen taken from sample (i) after incubation with the microbeads, did not form substantial particulates as observed by the microscope (Fig. 1A). In addition, analysis of the image obtained from this specimen revealed that the average size of the particulates formed by complexing between D-dimer and the microbeads is 21.6±1.8µm² (Fig. 2A).

A microscope specimen taken from sample (ii) containing intermediate levels of D-dimer produced aggregates visible by the microscope (Fig. 1B). In addition, analysis of the image obtained from this specimen revealed a shift in the distribution of the particulates size, with an average particulate size of 48.3±27.2µm² (Fig. 2B).

Finally, a specimen taken from sample (iii) containing high levels of D-dimer produced aggregates also visible by the microscope (Fig. 1C) and analysis of the image obtained from this specimen revealed an additional shift in the distribution of the particulate size (as compared to Fig. 2B), with an average particulate size of 156±155µm² (Fig. 2C).

These results obtained by detection of aggregates size distribution correlate with the levels of D-dimer in the tested samples.

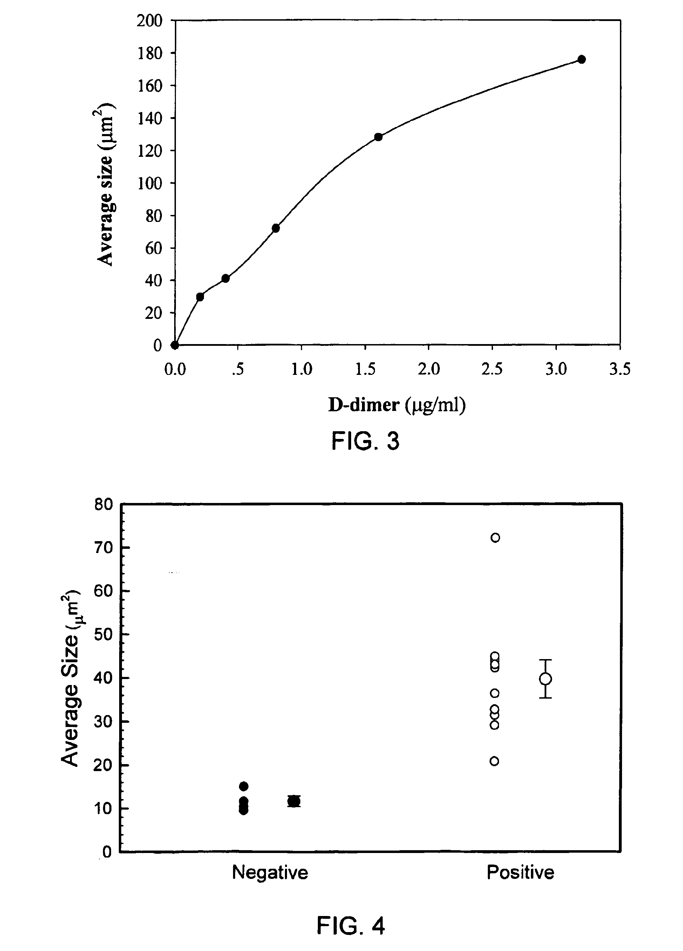

A titration curve of D-dimer concentrations in plasma samples was also determined. In particular, plasma samples containing different concentrations of D-dimer were incubated with anti-D-dimer antibody-coated beads for 1 minute after which specimens from the different samples were analyzed by the use of light microscope. Fig. 3 presents the titration curve obtained immediately after incubation period terminated and shows that there is a direct correlation between the D-dimer concentration in the samples and the average size of the aggregates formed as a result of complexing between D-dimer molecules present in the sample and the microbeads with which the sample was incubated. These results suggest the use of the method of the invention not only for qualitative determination but also as an analytical tool for quantitative determinations.

### Example 1.2

HIT syndrome results from an immune response to complex of heparin and platelet factor 4 (PF-4), which is located on the surface of platelet membrane, in some patients while treated by heparin. The result of this response is an immune mediated thrombocytopenia, or, in fewer cases, also thrombosis of the skin or other organs. In the following assay beads coated with heparin and PF-4 are used, which interact with a patient's plasma. In the case of a positive response, aggregates of beads are captured.

To this end, a HIT kit of Diamed (DiaMed, Cressier, Switzerland) was used in this assay and operated according to manufacturer's instructions in order to determine positive and negative samples. In general, plasma samples were mixed with ID-PaGIA polymer particles, at a ratio of 5:1, and incubated at room temperature for 5 minutes. Specimens from each sample were obtained for further analysis as described above.

Fourteen plasma samples were tested, 4 of which were negative and 10 positive according to Diamed kit. The average size of the particulates obtained by the image analyzer is presented in Fig. 4, which shows that the average size of the particulates in the negative control group was 11.6±1.2µm² while in the positive group 39.7±4.4 µm². Unpaired student-t test analysis of the data demonstrated a significant difference between the negative control group and the positive group of p<0.002.

### Example 2

The method of the invention was also applied for typing of blood groups. Accordingly, blood samples were taken from blood donors (with unknown blood groups). The blood samples were treated with an anti-coagulating agent (EDTA) and diluted (100 times) in a blood dilution buffer (0.9 % NaCl and 2% bovine albumine). Drops (10µl) of the diluted blood were placed on slides pre-coated with an antibody. Coating was achieved either by placing anti-group A or anti-group B antibodies on the slide (10µl of antibody at a concentration of 0.2 to 0.5 mg/mL and allowing the slide to air dry.

In an alternative procedure, the blood samples were treated with an anti-coagulating agent (EDTA) and diluted (50 times) in a blood dilution buffer, the diluted blood samples (5µl) were then mixed with anti-group A or anti-group B antibodies (5µl; 0.2 to 0.5 mg/mL) and each drop of the mixed sample was placed on a slide.

In each case a thin layer of the tested mixture was created before analysis.

As control, anti-coagulated blood samples were placed on an uncoated slide and without the presence of anti-group A or anti-group B antibodies. As a further control, blood samples of a known blood group were mixed with antibodies to other blood groups (e.g. blood group A was mixed with antibodies to blood group B).

The blood samples (either the control or samples mixed with the antibodies) were incubated for 15 seconds and then a cover slip was placed on the sample drop to form a thin layer of the sample (without direct contact with the slide, e.g. at a distanced of 0.5-1.0 mm from the slide). The covered samples were then exposed to ten light presses directed to the center of the blood drop and an optical image was obtained by the use of a CCD camera connected to an Image analyzer (Galai)

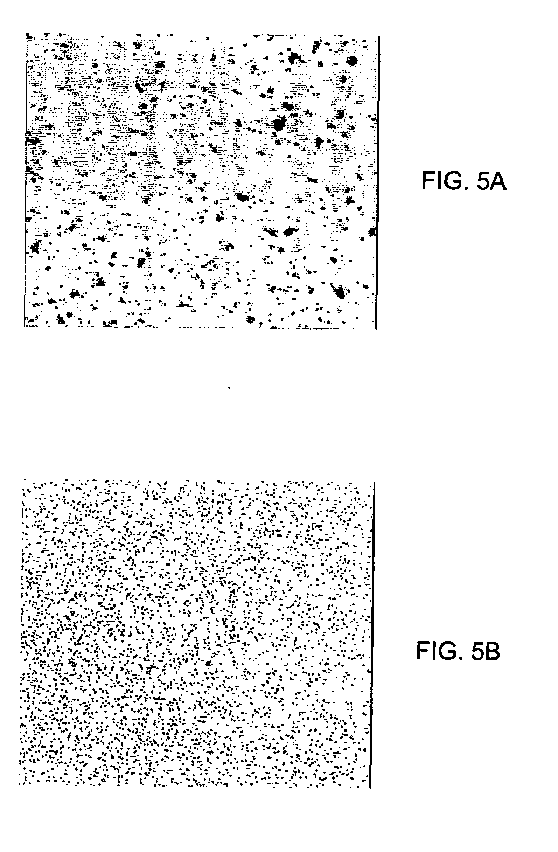

Fig. 5A shows an optical image of a negative response and in this particular case, a response between blood group A and anti-B antibodies is shown. Fig. 5B shows a response between blood group A and anti-A antibodies, an image of a positive response, which is exhibited by the formation of visible particulates (aggregates) as a result of association between the antibody carrying two capturing agent and the blood cell carrying multiple copies of the corresponding antigen.

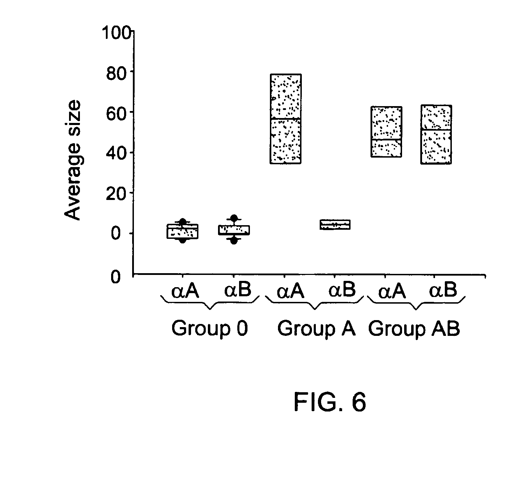

The average size and surface coverage of the aggregates on the slide was also determined. Table 1 and Fig. 6 presents the results, from which the blood group was derived.

**Table 1: Image analysis of blood groups**

| **Antiserum** | **Average size (µm²)** | **Surface coverage (%)** | **Blood group** |
|---|---|---|---|
| A | 64.9 | 4.1 | |
| | | | A |
| B | 22.7 | 1.9 | |
| | | | |
| A | 19.2 | 1.7 | |
| | | | B |
| B | 83.9 | 4.6 | |
| | | | |
| A | 68.5 | 5.4 | |
| | | | AB |
| B | 87.9 | 8.1 | |
| | | | |
| A | 17.1 | 1.2 | |
| | | | O |
| B | 16.7 | 2.3 | |

## Claims

1. A method for detecting an analyte in a fluid sample comprising:
(a) mixing said fluid sample with a reagent comprising a capturing agent which is a first member of a binding couple that can bind to an analyte, the analyte being a second member of the binding couple, such that if the analyte is present in the fluid sample, particulates of the binding couple are formed;
(b) treating said mixture so as to form on a solid substrate a monolayer of said particulates, if formed as a result of said mixing;
(c) obtaining an optical image of the monolayer; and
(d) analyzing said optical image so as to determine therefrom the absence or presence of particulates formed as a result of the association between the binding couple, the presence of particulates in the sample indicating the presence of said analyte in the sample; or to determine from said image at least one parameter of said particulates.

2. The method of Claim 1, wherein said analyte comprises at least two recognitions sites and said capturing agent comprises at least two capturing moieties, such that particulates of binding couples are formed by association of a recognition site of said analyte with a capturing moiety of said capturing agent.

3. The method of Claim 1 or 2, wherein said parameter is selected from: particulate size; size distribution of the particulates; particulates' count; shape of the particulates; or spatial distribution of the particulates.

4. The method of any one of Claims 1 to 3, wherein said image is a magnified image of said the monolayer of said mixture.

5. The method of Claim 4, wherein said magnification is achieved by the use of a light microscope lens.

6. The method of any one of Claims 1 to 5, wherein said analyte is a particle comprising on its surface two or more copies of a recognition site with which a capturing agent can associate, thereby forming particulates of said binding couple.

7. The method of Claim 6, wherein said particle is a cell or a microorganism presenting on their surface said recognition sites.

8. The method of Claim 7, wherein the recognition site is an antigen and said capturing agent is an antibody having affinity to said antigen.

9. The method of any one of Claims 1 to 5, wherein said reagent comprises microbeads having a sensing interface, the sensing interface carrying two or more copies of a capturing moiety such that if said analyte is present in the fluid sample, particulates of binding couples are formed by association of said capturing moieties on said microbead with recognition sites of said analyte.

10. The method of Claim 9, wherein said sensing interface carries two or more copies of a same capturing moiety.

11. The method of Claim 9, wherein said sensing interface carries two or more copies of different capturing moieties.

12. The method of any one of Claims 10 to 12, wherein said microbeads are affinity microbeads.

13. The method of Claim 13, wherein said microbeads are immunobeads.

14. The method of any preceding claim, wherein the monolayer is a uniform layer of particulates, and wherein the uniform layer of particulates comprises essentially no overlaying of one binding couple on top of another binding couple and comprises substantially only one particulate or aggregate at the vertical dimension of the layer.

## Patentansprüche

1. Verfahren zum Nachweisen eines Analyten in einer flüssigen Probe, das Folgendes umfasst:
(a) Mischen von genannter flüssiger Probe mit einem Reagenz, das ein Einfangmittel umfasst, das ein erster Teil eines Bindungspaars ist, der zu einem Analyten binden kann, wobei der Analyt ein zweiter Teil des Bindungspaars ist, sodass beim Vorliegen des Analyten in der flüssigen Probe Partikel des Bindungspaars gebildet werden;
(b) Behandeln von genannter Mischung, um auf einem festen Substrat eine Monoschicht von genannten Partikeln zu bilden, falls sie infolge von genanntem Mischen gebildet werden;
(c) Erhalten eines optischen Bilds der Monoschicht; und
(d) Analysieren von genanntem optischen Bild, um davon die Gegenwart oder Abwesenheit von Partikeln, die infolge der Verbindung zwischen dem Bindungspaar gebildet wurden, zu bestimmen, wobei die Gegenwart von Partikeln in der Probe die Gegenwart von genanntem Analyten in der Probe anzeigt; oder um aus dem genannten Bild mindestens einen Parameter von genannten Partikeln zu bestimmen.

2. Verfahren nach Anspruch 1, worin genannter Analyt mindestens zwei Erkennungsstellen umfasst und genanntes Einfangmittel mindestens zwei Einfangeinheiten umfasst, sodass Partikel von Bindungspaaren durch Verbindung einer Erkennungsstelle von genanntem Analyten mit einer Einfangeinheit von genanntem Einfangmittel gebildet werden.

3. Verfahren nach Anspruch 1 oder 2, worin genannter Parameter aus Folgenden ausgewählt ist: Partikelgröße; Größenverteilung der Partikel; Zahl der Partikel; Form der Partikel; oder räumliche Verteilung der Partikel.

4. Verfahren nach einem der Ansprüche 1 bis 3, worin genanntes Bild ein vergrößertes Bild der genannten Monoschicht von genannter Mischung ist.

5. Verfahren nach Anspruch 4, worin genannte Vergrößerung durch die Verwendung eines Lichtmikroskopobjektivs erzielt wird.

6. Verfahren nach einem der Ansprüche 1 bis 5, worin genannter Analyt ein Teilchen ist, das auf seiner Oberfläche zwei oder mehr Kopien einer Erkennungsstelle umfasst, mit der sich ein Einfangmittel verbinden kann, wodurch Partikel von genanntem Bindungspaar gebildet werden.

7. Verfahren nach Anspruch 6, worin genanntes Teilchen eine Zelle oder ein Mikroorganismus ist, die auf ihrer Oberfläche genannte Erkennungsstellen präsentieren.

8. Verfahren nach Anspruch 7, worin die Erkennungsstelle ein Antigen ist und genanntes Einfangmittel ein Antikörper mit Affinität zum genannten Antigen ist.

9. Verfahren nach einem der Ansprüche 1 bis 5, worin genanntes Reagenz Mikrobeads mit einer erkennenden Grenzfläche umfasst, wobei die erkennende Grenzfläche zwei oder mehr Kopien einer Einfangeinheit trägt, sodass beim Vorliegen von genanntem Analyten in der flüssigen Probe Partikel von Bindungspaaren durch Verbindung von genannten Einfangeinheiten auf genanntem Mikrobead mit Erkennungsstellen von genanntem Analyten gebildet werden.

10. Verfahren nach Anspruch 9, worin genannte erkennende Grenzfläche zwei oder mehr Kopien einer gleichen Einfangeinheit trägt.

11. Verfahren nach Anspruch 9, worin genannte erkennende Grenzfläche zwei oder mehr Kopien von verschiedenen Einfangeinheiten trägt.

12. Verfahren nach einem der Ansprüche 10 bis 12, worin genannte Mikrobeads Mikrobeads fiir Affinität sind.

13. Verfahren nach Anspruch 13, worin genannte Mikrobeads Immunobeads sind.

14. Verfahren nach einem vorstehenden Anspruch, worin die Monoschicht eine gleichmäßige Schicht von Partikeln ist und worin die gleichmäßige Schicht von Partikeln im Wesentlichen kein Überlagern von einem Bindungspaar auf einem anderen Bindungspaar umfasst und größtenteils nur ein Partikel oder Aggregat in der vertikalen Dimension der Schicht umfasst.

## Revendications

1. Méthode de détection d'un analyte dans un échantillon de fluide comprenant :
(a) le mélange dudit échantillon de fluide avec un réactif comprenant un agent de capture qui est un premier élément d'un couple de liaison pouvant se lier à un analyte, l'analyte étant un deuxième élément du couple de liaison, de sorte que si l'analyte est présent dans l'échantillon de fluide, des particules du couple de liaison se forment ;
(b) le traitement dudit mélange de manière à former, sur un substrat solide, une monocouche desdites particules, si elles sont formées suite audit mélange;
(c) l'obtention d'une image optique de la monocouche ; et
(d) l'analyse de ladite image optique afin de déterminer à partir de celle-ci l'absence ou la présence de particules formées suite à l'association entre le couple de liaison, la présence de particules dans l'échantillon indiquant la présence dudit analyte dans l'échantillon ; ou déterminer à partir de ladite image au moins un paramètre desdites particules.

2. Méthode selon la revendication 1, dans laquelle ledit analyte comprend au moins deux sites de reconnaissance et ledit agent de capture comprend au moins deux motifs de capture, de sorte que des particules des couples de liaison se forment par association d'un site de reconnaissance dudit analyte avec un motif de capture dudit agent de capture.

3. Méthode selon la revendication 1 ou 2, dans laquelle ledit paramètre est choisi parmi : la taille de particule, la granulométrie des particules, le compte des particules, la forme des particules ou la répartition spatiale des particules.

4. Méthode selon l'une quelconque des revendications 1 à 3, dans laquelle ladite image est une image grossie de ladite monocouche dudit mélange.

5. Méthode selon la revendication 4, dans laquelle ledit grossissement est réalisé au moyen d'une lentille de microscope optique.

6. Méthode selon l'une quelconque des revendications 1 à 5, dans laquelle ledit analyte est une particule comprenant sur sa surface deux copies ou plus d'un site de reconnaissance avec lequel un agent de capture peut s'associer, formant ainsi des particules dudit couple de liaison.

7. Méthode selon la revendication 6, dans laquelle ladite particule est une cellule ou un microorganisme présentant lesdits sites de reconnaissance sur sa surface.

8. Méthode selon la revendication 7, dans laquelle le site de reconnaissance est un antigène et ledit agent de capture est un anticorps ayant une affinité vis-à-vis dudit antigène.

9. Méthode selon l'une quelconque des revendications 1 à 5, dans laquelle ledit réactif comprend des microbilles ayant une interface de détection, l'interface de détection portant deux copies ou plus d'un motif de capture de sorte que si ledit analyte est présent dans l'échantillon de fluide, des particules des couples de liaison se forment par association desdits motifs de capture sur ladite microbille avec des sites de reconnaissance dudit analyte.

10. Méthode selon la revendication 9, dans laquelle ladite interface de détection porte deux copies ou plus d'un même motif de capture.

11. Méthode selon la revendication 9, dans laquelle ladite interface de détection porte deux copies ou plus de différents motifs de capture.

12. Méthode selon l'une quelconque des revendications 10 à 12, dans laquelle lesdites microbilles sont des microbilles d'affinité.

13. Méthode selon la revendication 13, dans laquelle lesdites microbilles sont des immunobilles.

14. Méthode selon l'une quelconque des revendications précédentes, dans laquelle la monocouche est une couche uniforme de particules, et dans laquelle la couche uniforme de particules ne comprend essentiellement pas de recouvrement d'un couple de liaison par-dessus un autre couple de liaison et comprend substantiellement une seule particule ou un seul agrégat à la dimension verticale de la couche.
